# EUROPEAN PATENT APPLICATION

(11) **EP 4 005 548 A1**
(43) Date of publication of application: **01.06.2022**
(21) Application number: 20843280.7
(22) Date of filing: 27.07.2020
(51) Int. Cl.: A61K 8/365, A61K 8/34, A61K 8/64, A61Q 5/04

(54) **HAIR TREATMENT COMPOSITION, METHOD FOR OBTAINING SAME, USE OF THE COMPOSITION AND HAIR TREATMENT METHOD**

(30) Priority: 25.07.2019 BR 102019015359
(71) Applicant: Chemyunion Ltda, 18087-101 Sorocaba, SP (BR)
(72) Inventor: NOGUEIRA, Cecília, 18085-844 Sorocaba/SP (BR); DE CAMARGO JUNIOR, Flávio Bueno, 18013-004 Sorocaba/SP (BR)
(74) Representative: Grünecker Patent- und Rechtsanwälte PartG mbB
(86) International application number: PCT/BR2020/050291
(87) International publication number: WO 2021/012028

(57) **Abstract**

The present invention describes a composition for hair treatments, as well as a process for obtaining the same, comprising a molecule of glyoxyloyl protein, more specifically, hydrolyzed wheat protein and sericin. Specifically, the composition promotes hair straightening, without damaging the hair fibers, quickly and safely. The present invention is pertained to the fields of Chemistry and Cosmetics.

## Description

### Field of the Invention

The present invention describes a composition for hair treatments comprising glyoxyloyl molecule obtained from the reaction of glyoxylic acid with proteins, more specifically, hydrolyzed wheat proteins and sericin, wherein the composition promotes hair straightening.

### Background of the Invention

Hair is basically made up of a protein: α-keratin; composed of 4 polypeptide chains formed by a series of amino acids and sulfur. One of the amino acids present and most related to the structure of the thread is cysteine, responsible for cysteine bonds. The intramolecular bonds between amino acids in the same chain promote the support of the keratin structure. Among the types of interactions, we have weak ones such as hydrogen bonds and saline ones, and strong ones represented by disulfide bonds. The weak ones are broken in the simple act of wetting the hair.

Currently, there is a great demand for methods of hair straightening, and therefore it is required to break or attack the sulfur bonds contained in cysteine. The current methods use physicochemical techniques, such as the dryer and the piastre ("flat iron") and "hot comb" (hot straightening comb) which only last until the next wash. These methods require the hair to be pre-wetted, so that the hydrogen bonds break in the keratin hydrolysis process, thus allowing the temporary opening of its helical structure. Hence, the strand become smooth. Rapid dehydration with the dryer maintains the smooth shape of the rod. The application of the hot hair straightener molds the cuticle cells (scales), as if flattening them parallel to the rod. The strand acquires a smooth and shiny appearance, as it reflects more incident light. The search for straight hair is constant, and for those who have frizzy or curly hair and want a new shape, it is necessary to break or attack the sulfur bonds contained in cysteine.

The definitive straightening methods, on the other hand, present chemical processes that promote the breaking of keratin disulfide bonds. The possibility of interconversion between the oxidized (RSSR) and reduced (RSH) forms of cysteine is that it allows you to straighten frizzy hair or curl straight hair. In general, the reaction occurs with formulas containing some known chemical agents, such as: sodium, lithium and potassium hydroxide, guanidine hydroxide (calcium hydroxide plus guanidine carbonate), bisulfites and ammonia thioglycolate or ethanolamine, and occur at a highly alkaline pH.

Another chemical agent, often used for hair relaxation and straightening processes is formaldehyde (simpler aldehyde, molecular formula H₂CO), because, in addition to the lower cost, the application of this agent occurs through a fast process that leaves the strands with intense shine. The frequent use of formaldehyde for hair straightening presents some problems, the biggest one being the volatility of the agent. Once heated, a large amount of the agent can be inhaled by both the person applying and the person undergoing the treatment. It is postulated that formaldehyde binds to cuticle proteins and hydrolyzed amino acids in the keratin solution, forming a hardening film along the hairline, waterproofing it and keeping it rigid and smooth. The strand becomes susceptible to breakage as a result of normal everyday traumas, such as combing and pinning the hair.

A compound of lower molecular weight containing the organic functions, carboxylic acid and aldehyde, is glyoxylic acid. This combination promotes excellent aldehyde reactivity and low volatility, making it safer in handling, unlike other low molecular weight aldehydes that are volatile or even gases such as formaldehyde, easily absorbed in breathing. As a compound with a small molecular structure, it allows the aldehyde to have access to the hair reactive centers. However, despite these characteristics, its use at the temperature of the dryer and the hair straightener are some of the variables that can interfere with the maintenance of the color of dyed hair that undergoes relaxation processes.

Thus, from what appears from the researched literature, no documents were found anticipating or suggesting the teachings of the present invention, so that the solution proposed herein has novelty and inventive step compared to the state of the art.

Therefore, it is required to develop methods of hair treatment that promote the straightening of the hair in a quick and definitive way, but that does not present a risk to those who use or manipulate the product, as in existing processes that use molecules of low molecular weight and high aldehyde reactivity.

### Summary of the Invention

Thus, the present invention solves the problems of the state of the art from compositions for hair treatment comprising the protein glyoxyloyl molecule, more specifically, hydrolyzed wheat protein and sericin which has low volatility and excellent aldehyde reactivity, promoting the straightening of the strands, without causing damage to the hair fibers, and in a safer way compared to other low molecular weight compounds for hair straightening.

In a first object, the present invention provides a composition for hair treatment comprising from 5% to 20% by weight of glyoxyloyl hydrolyzed wheat protein and sericin.

In a second object the present invention provides a method of hair treatment comprising a step of applying to hair a composition for hair treatment comprising from 5% to 20% by weight of glyoxyloyl hydrolyzed wheat protein and sericin.

In a third object, the present invention presents a process for obtaining a composition for hair treatment comprising the steps of:

a) React glyoxylic acid with an alcohol, in which the reaction occurs under reflux in an acidic medium for 2 to 4 hours, forming an ethyl diethoxyacetate;

b) React ethyl diethoxyacetate with at least one protein selected from the group consisting of hydrolyzed wheat protein and sericin, in which the reaction occurs in an acidic medium for 1 hour in a temperature range between 70 °C to 85 °C, forming glyoxyloyl hydrolyzed wheat protein and sericin;

c) Mix 5% to 20% by weight of the final composition of glyoxyloyl hydrolyzed wheat protein and sericin with at least one cosmetically acceptable excipient.

In a fourth object, the present invention presents the use of the composition for hair treatment to promote the straightening of the strands and/or maintenance of the mechanical properties of the strands, in which the composition for hair treatment comprising from 5% to 20% by weight of glyoxyloyl hydrolyzed wheat protein and sericin.

These and other objects of the invention will be immediately appreciated by those skilled in the art and will be described in detail below.

### Brief Description of the Drawings

The following figures are presented:
Figure 1 shows locks from group 1 (control), before (A) and after (B) received treatment with thermal straightening process.
Figure 2 shows locks from group 2, before (A) and after (B) received treatment with chemical straightening process (straightening cream with Ammonium Thioglycolate).
Figure 3 shows locks from group 3, before (A) and after (B) received treatment with chemical straightening process (straightening cream with Guanidine Hydroxide).
Figure 4 shows locks from group 4, before (A) and after (B) received treatment with chemical straightening process (straightening cream with Glyoxyloyl Hydrolyzed Wheat Protein and Sericin).
Figure 5 shows the values (gf) of maximum force resisted by the hair strand until its breakage.

### Detailed Description of the Invention

The present invention presents compositions for hair treatment, the process of obtaining the same comprising a glyoxyloyl molecule obtained from hydrolyzed wheat proteins and sericin, and its use for straightening the hair, without causing damage to the hair fibers, and more reliably than other low molecular weight molecules for that purpose.

The glyoxyloyl molecule obtained from hydrolyzed wheat proteins and sericin, has low molecular weight, high aldehyde reactivity and low volatility. Thus, the present invention promotes the desired hair straightening, in a safer way than other compounds already on the market, since it is not volatile, and, therefore, it is not toxic for those who handle it.

The wheat amino acid has plant origin and the amino acid profile of the wheat protein is characterized by the high concentration of glutamine (30%) and proline (10%). Sericin, on the other hand, comes from the cocoon of silkworms, an animal source, and consists mainly of amino acids: serine (33%), aspartic acid (20%), glycine (14%) and threonine (8%).

In a first object, the present invention provides a composition for hair treatment comprising from 5% to 20% by weight of glyoxyloyl hydrolyzed wheat protein and sericin.

In one embodiment, the composition for hair treatment additionally comprises from 5% to 15% by weight of cetostearyl alcohol and/or cetostearyl alcohol ethoxylated 20EO.

In one embodiment, the composition for hair treatment further comprises from 0.01% to 5% by weight of a cosmetically acceptable excipient. A non-exhaustive list of cosmetically acceptable excipients comprises emulsifiers, preservatives and solubilizers.

In one embodiment, the cosmetically acceptable excipient is selected from at least one of the group consisting of: Behentrimonium Chloride and/or Cetearyl Alcohol and/or Astrocaryum Murumuru Seed Butter; Mineral oil; Vaseline; Phenoxyethanol and Parabens; Glycerin.

In one embodiment, the composition for hair treatment comprises from 10% to 15% by weight of glyoxyloyl hydrolyzed wheat protein and sericin.

In a second object the present invention provides a method of hair treatment comprising a step of applying to hair a composition for hair treatment comprising from 5% to 20% by weight of glyoxyloyl hydrolyzed wheat protein and sericin.

In one embodiment, the application step lasts from 10 minutes to 30 minutes.

In one embodiment, hair is washed and dried prior to the application step.

In a third object, the present invention presents a process for obtaining a composition for hair treatment comprising the steps of:

a) React glyoxylic acid with an alcohol, in which the reaction occurs under reflux in an acidic medium for 2 to 4 hours, forming an ethyl diethoxyacetate;

b) React ethyl diethoxyacetate with at least one protein selected from the group consisting of hydrolyzed wheat protein and sericin, in which the reaction occurs in an acidic medium for 1 hour in a temperature range between 70 °C to 85 °C, forming hydrolyzed wheat protein glyoxyloyl and sericin;

c) Mix 5% to 20% by weight of the final composition of glyoxyloyl hydrolyzed wheat protein and sericin with at least one cosmetically acceptable excipient.

In a fourth object, the present invention presents the use of the composition for hair treatment to promote the straightening of the strands and/or maintenance of the mechanical properties of the strands, in which the composition for hair treatment comprising from 5% to 20% by weight of glyoxyloyl hydrolyzed wheat protein and sericin.

The present invention has the advantage of promoting the relaxation and realignment of the hair strands, with proven effectiveness in reducing damage to the strands, protecting the hair against breakage, increasing the cross-sectional area of the hair fiber, promoting a smooth effect long lasting and improves shine through a highly efficient protein complex.

### Examples

The examples shown herein are intended only to exemplify one of the numerous ways to carry out the invention, however without limiting its scope.

### Preparation of Glyoxyloyl from Hydrolyzed Wheat Protein and Sericin

The synthesis of the glyoxyloyl product from hydrolyzed wheat protein and sericin occurred in 3 synthetic steps. In the first synthetic step, glyoxylic acid was converted into ethyl diethoxyacetate, through the reaction of esterification and acetalization with ethanol in a molar ratio of 1:9 (glyoxylic acid:ethanol) in the presence of an acid catalyst (H₃PO₄). The reaction medium was refluxed for a period of 2 to 4 hours. After this period, excess ethanol was removed by distillation. In the second synthetic step, hydrolyzed wheat protein and sericin were added to the same reaction medium, which have amino and hydroxyl functional groups in their molecular structure, which quickly react with ethyl diethoxyacetate through transesterification and amidation reactions in the presence of H₃PO₄, for a period of 1 hour at a temperature range between 70 °C and 85 °C. In the last step, the decetalization process occurred due to the addition of water to the reaction medium and removal of ethanol by distillation, leading to the formation of a solution containing 74% to 85% (w/w) of glyoxyloyl hydrolyzed wheat protein and sericin, 15% to 25% (w/w) of water and 0.2% to 0.8% (w/w) of residual sericin. Process yield: 70% (w/w).

### Comparative evaluation of the straightening effect and mechanical properties of hair locks.

Tests were performed to evaluate the effectiveness of Glyoxyloyl Hydrolyzed Wheat Protein and Sericin, in the straightening and maintenance of the mechanical properties (strength) of hair fibers, compared to smoothers based on Ammonium Thioglycolate, Guanidine Hydroxide and a control group (thermal straightening).

### Formulations evaluated

To carry out the study, formulations of straightening creams were developed, plus Ammonium Thioglycolate (Straightening Cream + Neutralizing Lotion - Tables 1 and 2), Guanidine Hydroxide (Calcium Hydroxide + Guanidine Carbonate - Tables 3 and 4 ) and Glyoxyloyl Hydrolyzed Wheat Protein and Sericin (Table 5).

**Table 1: Straightening Cream with Ammonium Thioglycolate.**

| **STRAIGHTENING CREAM (Ammonium Thioglycolate 8.26%)** | |
|---|---|
| **Components** | **(% w/w)** |
| Cetearyl Alcohol (and) Cetostearyl Alcohol Ethoxylated 20EO | 10.00 |
| Behentrimonium Chloride (and) Cetearyl Alcohol (and) Astrocaryum Murumuru Seed Butter | 2.00 |
| Mineral Oil | 5.00 |
| Vaseline | 5.00 |
| Phenoxyethanol and Parabens | 0.50 |
| Glycerin | 3.00 |
| Ammonium Thioglycolate (59%) | 14.00 |
| Sodium metabisulfite | 0.30 |
| Deionized water q.s.p. | 100.00 |
| Ammonium hydroxide (25%) q.s.p. | pH 8.5 - 9.5 |

**Table 2: Neutralizing lotion, for the straightening cream plus Ammonium Thioglycolate.**

| **NEUTRALIZING LOTION (Hydrogen peroxide 2.1%)** | |
|---|---|
| **Components** | **(% w/w)** |
| Cetostearyl Alcohol | 3.20 |
| DTPA Pentasodium solution 40.2% | 1.00 |
| Phenacetin | 0.10 |
| Ethidronic acid | 0.06 |
| Phosphoric acid (85%) | 0.10 |
| Sodium lauryl ether sulfate (27%) | 2.00 |
| Hydrogen peroxide (29%) | 7.25 |
| Polyquaternium-7 | 1.00 |
| Deionized water q.s.p. | 100.00 |
| Phosphoric Acid (85%) q.s.p. | pH 2.5 - 3.5 |

**Table 3: Straightening Cream plus Calcium Hydroxide.**

| **STRAIGHTENING CREAM (Calcium Hydroxide 6.0%)** | |
|---|---|
| **Components** | **(% w/w)** |
| Cetearyl Alcohol (and) Cetostearyl Alcohol Ethoxylated 20EO | 10.00 |
| Behentrimonium Chloride (and) Cetearyl Alcohol (and) Astrocaryum Murumuru Seed Butter | 2.00 |
| Mineral Oil | 5.00 |
| Vaseline | 5.00 |
| Phenoxyethanol and Parabens | 0.50 |
| Glycerin | 3.00 |
| Calcium Hydroxide | 6.00 |
| Deionized water q.s.p. | 100.00 |

**Table 4: Activating fluid with Guanidine Carbonate, for mixing with straightening cream plus Calcium Hydroxide, to obtain a formulation with Guanidine Hydroxide.**

| **ACTIVATING FLUID (Guanidine Carbonate 25.0%) Components** | **(% w/w)** |
|---|---|
| | |
| DTPA Pentasodium solution 40.2% | 0.50 |
| Polyquaternium-7 | 1.00 |
| Guanidine Carbonate | 25.00 |
| Deionized water q.s.p. | 100.00 |

**Table 5: Straightening Cream plus Glyoxyloyl Hydrolyzed Wheat Protein and Sericin.**

| **STRAIGHTENING CREAM (Glyoxyloyl Hydrolyzed Wheat Protein and Sericin 10.0%)** | |
|---|---|
| **Components** | **(% w/w)** |
| Cetearyl Alcohol (and) Cetostearyl Alcohol Ethoxylated 20EO | 10.00 |
| Behentrimonium Chloride (and) Cetearyl Alcohol (and) Astrocaryum Murumuru Seed Butter | 2.00 |
| Mineral Oil | 5.00 |
| Vaseline | 5.00 |
| Phenoxyethanol and Parabens | 0.50 |
| Glycerin | 3.00 |
| Glyoxyloyl Hydrolyzed Wheat Protein and Sericin (70%) | 14.30 |
| Deionized water q.s.p. | 100.00 |

### Locks of Hair

The evaluations were carried out in triplicate, in virgin brown locks of hair (length of 30 cm and average weight of 4 g), extra tight curly type, supplied by the company De Meo Brothers - Inc.

### Pre-treatment of locks

The locks were washed with anti-residue shampoo and dried naturally (24±1h) before treatment.

### Application of formulations

The locks in group 1 were undergone to a thermal straightening process with piastre (10 passes at 200°C). After straightening, the locks were washed with standardized amounts of shampoo and conditioner. After applying the conditioner, the locks were rinsed and dried naturally (control group).

For the treatment of locks in group 2, a standardized amount of straightening cream plus Ammonium Thioglycolate was applied to dry hair. After application, the product remained acting on the hair for 18 minutes. After the action time, the locks were rinsed with warm water, until the product was completely removed. Then, the hair was neutralized with a neutralizing lotion, to stop the action of the product on the hair. After neutralization, the locks were rinsed with warm water and washed with standardized amounts of shampoo and conditioner. To finish the process, the locks were rinsed and dried naturally.

For the treatment of locks in group 3, a standardized amount of straightening cream was applied to dry hair plus a mixture of Calcium Hydroxide and Guanidine Carbonate (1:0.25). After application, the product remained acting on the hair for 18 minutes. Then, the locks were rinsed with warm water until the product was completely removed. After rinsing, the locks were washed with standardized amounts of shampoo and conditioner. To finish the process, the locks were rinsed and dried naturally.

For the treatment of locks in group 4, after washing with shampoo and removing excess water from the locks with a towel, a standardized amount of straightening cream added with Glyoxyloyl Hydrolyzed Wheat Protein and Sericin was applied homogeneously in damp hair. After application, the product remained acting on the hair for 20 minutes. Next, a brushing process with the dryer was carried out, followed by thermal straightening with a piastre (10 passes at 200°C). After thermal straightening, the locks were left to rest for 15 minutes and then rinsed with water. To complete the process, the locks received the application of a standardized amount of conditioner and were dried with a dryer.

### Straightening effect of the object of study formulations.

After applying the straightening processes on the locks, the effectiveness of the straightening effect of the formulations were evaluated.

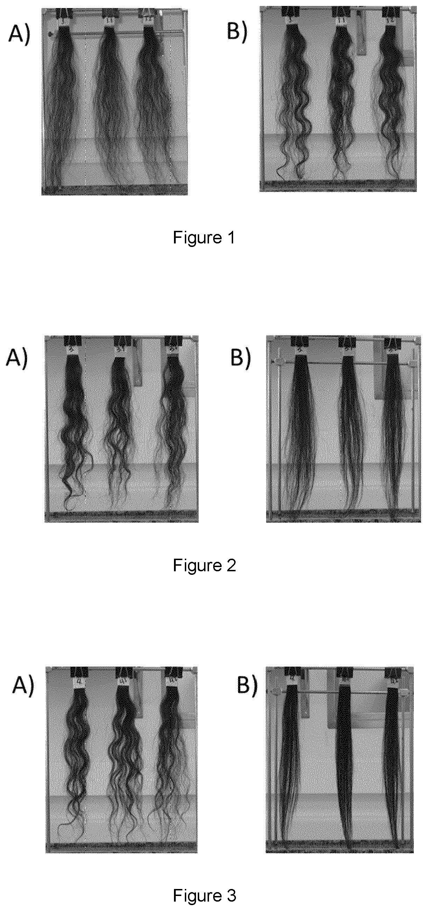

Through the subjective evaluation by analyzing the images captured before and after the treatments, it was possible to verify that the locks in group 1 (control) that underwent only the thermal straightening process (dryer and piastre), not the presented satisfactory straightening (Figure 1).

The locks of groups 2, 3 and 4 that underwent a straightening process with the addition of Ammonium Thioglycolate formulations, or Guanidine Hydroxide or Glyoxyloyl Hydrolyzed Wheat Protein and Sericin, respectively, showed satisfactory straightening after the treatments . It is not possible to observe significant differences between the treatments of locks in groups 2, 3 and 4 (Figures 2, 3 and 4 respectively).

Finally, sensory tests were performed with the composition of the present invention, wherein the vast majority of people had a highly positive feedback.

### Evaluation of Mechanical Properties

The evaluation of mechanical properties was performed using the universal machine for mechanical testing, model DL 2000 (EMIC), equipped with a load cell with a capacity of 10 kgf, with traction speed rates of 10 mm/min. For the study, a claw with simple grip and fastening by smooth jaws and a geometric tension attenuator auger was used.

For the mechanical properties, the mean values of maximum force (gf) held out by 200 hairs of each of the 4 groups object of study were evaluated. For the test, the threads were grouped into 10 samples with 20 threads each, fixed at both ends (25 cm apart) with hot resin-based glue. To certify that the samples were prepared effectively, preliminary tests were carried out to ensure that the strand breakage would occur between the fixation points and not in the hot glue fixed region.

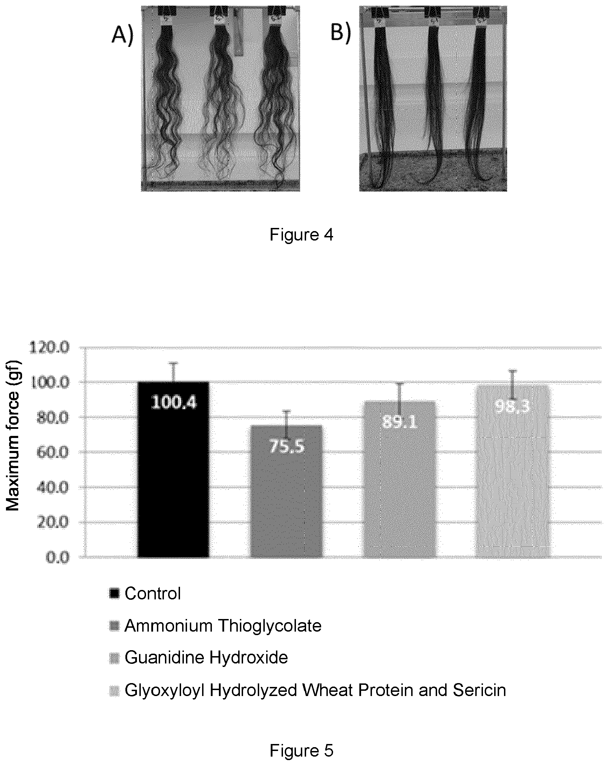

By the results it was possible to observe that the fibers of group 1 (control), which did not undergo chemical straightening, endured an average force of 100.4 gf per fiber evaluated, before causing their rupture (Figure 5).

The group 2 locks that went through the straightening process with Ammonium Thioglycolate, withstood an average force of 75.5 gf, showing a reduction of 24.8% in the values of force required to cause the hair to break, compared to the fibers of the control group. The results indicate a decrease in hair mechanical strength after treatment (Figure 5).

The locks of group 3 that underwent a straightening process with Guanidine Hydroxide, resisted an average force of 89.1gf, showing a reduction of 11.2% in the values of force required to cause hair breakage, in comparison with the fibers of the control group. The results indicate a decrease in hair mechanical strength after treatment (Figure 5).

The locks of group 4 that underwent straightening with Glyoxyloyl Hydrolyzed Wheat Protein and Sericin, resisted an average force of 98.3gf, presenting a 2.1% reduction in the values of force necessary to cause the rupture of the strands, compared to the fibers of the control group. The results indicate that the straightening with Glyoxyloyl Hydrolyzed Wheat Protein and Sericin caused the least damage to the capillary resistance after treatment (Figure 5).

Those skilled in the art will appreciate the knowledge presented herein and may reproduce the invention in the presented modalities and in other variants and alternatives, covered by the scope of the following claims.

## Claims

1. Composition for hair treatment **comprising** from 5% to 20% by weight of a glyoxyloyl hydrolyzed wheat protein and sericin.

2. Composition for hair treatment, according to claim 1, further **comprising** from 5% to 15% by weight of cetearyl alcohol and/or ethoxylated cetostearyl alcohol 20EO.

3. Composition for hair treatment, according to any one of the preceding claims, **comprising** from 10% to 15% by weight of glyoxyloyl protein.

4. A method of hair treatment, **comprising** a step of applying to hair a composition for hair treatment according to any one of the preceding claims.

5. Method of hair treatment according to claim 4, **characterized in that** the application step lasts from 10 minutes to 30 minutes.

6. Process for obtaining the composition for hair treatment, according to any one of the claims 1 to 3, **comprising** the steps of:
a) React glyoxylic acid with an alcohol, in which the reaction occurs under reflux in an acidic medium for 2 to 4 hours, forming an ethyl diethoxyacetate;
b) React ethyl diethoxyacetate with at least one protein selected from the group consisting of hydrolyzed wheat protein and sericin, in which the reaction occurs in an acidic medium for 1 hour in a temperature range between 70 °C to 85 °C, forming glyoxyloyl hydrolyzed wheat protein and sericin;
c) Mix 5% to 20% by weight of the final composition of glyoxyloyl hydrolyzed wheat protein and sericin with at least one cosmetically acceptable excipient.

7. Use of the composition for hair treatment, according to any one of the claims 1 to 3, **characterized in that** it is to promote the straightening of the strands and/or maintenance of the mechanical properties of the strands.
